(19)

Europäisches
Patentamt
European
Patent Office
Office européen
des brevets

(11) **EP 3 893 249 A2**

(12) **EUROPEAN PATENT APPLICATION**

(43) Date of publication:
**13.10.2021 Bulletin 2021/41**

(51) Int Cl.:
**G16H 50/80** *(2018.01)*

(21) Application number: **21192800.7**

(22) Date of filing: **24.08.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.02.2021 CN 202110159528**

(71) Applicant: **Beijing Baidu Netcom
Science and Technology Co., Ltd.
Beijing 100085 (CN)**

(72) Inventors:
• **LIU, Ji
  Beijing 100085 (CN)**
• **DOU, Dejing
  Beijing 100085 (CN)**
• **HUANG, Jizhou
  Beijing 100085 (CN)**
• **LI, Qiaojun
  Beijing 100085 (CN)**

(74) Representative: **Nederlandsch Octrooibureau
P.O. Box 29720
2502 LS The Hague (NL)**

(54) **METHOD FOR PREDICTING EPIDEMICS**

(57) The present disclosure provides a method and apparatus for predicting an epidemic situation, a device, a storage medium, and a program product, relates to the field of artificial intelligence technology such as deep learning and smart medical care. A specific embodiment of the method includes: estimating a variable parameter of the epidemic situation in a preset area using a Markov Chain Monte Carlo (MCMC) method; acquiring a constant parameter of the epidemic situation in the preset area; constructing a transmission model based on the variable parameter and the constant parameter; and fitting, using the transmission model, to predict epidemic information of the preset area. This embodiment uses the transmission model and the MCMC method to predict the epidemic situation in the preset area, which improves an accuracy of epidemic prediction.

100

Estimating a variable parameter of the epidemic situation in a preset area using a Markov Chain Monte Carlo (MCMC) method — 101

Acquiring a constant parameter of the epidemic situation in the preset area — 102

Constructing a transmission model based on the variable parameter and the constant parameter — 103

Fitting, using the transmission model, to predict epidemic information of the preset area — 104

Fig. 1

EP 3 893 249 A2

**Description**

TECHNICAL FIELD

The present disclosure relates to the field of computer technology, in particular to the field of artificial intelligence technology such as deep learning and smart medical care, and more in particular to a method and apparatus for predicting an epidemic situation, a device, a storage medium, and a program product.

BACKGROUND

In recent years, large-scale epidemic situations occurred in a large area. Epidemic situations refer to the occurrence or development of epidemic diseases, and epidemic diseases may be severe infectious diseases occurred in a large area. The epidemic situations occurred suddenly, broke out in countries around the world, spread quickly across countries, and became global emergencies, causing huge impacts on people lives. To deal with the spread of epidemic diseases, the governments of many countries have adopted a series of measures.

Through the establishment of mathematical models, the epidemic situations can be analyzed and the spread trends of the epidemic situations can be effectively grasped.

SUMMARY

The present disclosure provides a method and apparatus for predicting an epidemic situation, a device, a storage medium, and a program product.

According to a first aspect of the present disclosure, a method for predicting an epidemic situation is provided, and the method includes: estimating a variable parameter of the epidemic situation in a preset area using a Markov Chain Monte Carlo (MCMC) method; acquiring a constant parameter of the epidemic situation in the preset area; constructing a transmission model based on the variable parameter and the constant parameter; and fitting, using the transmission model, to predict epidemic information of the preset area.

According to a second aspect of the present disclosure, an apparatus for predicting an epidemic situation is provided, and the apparatus includes: an estimation module configured to estimate a variable parameter of the epidemic situation in a preset area using a Markov Chain Monte Carlo (MCMC) method; an acquisition module configured to acquire a constant parameter of the epidemic situation in the preset area; a construction module configured to construct a transmission model based on the variable parameter and the constant parameter; and a fitting module configured to fit, using the transmission model, to predict epidemic information of the preset area.

According to a third aspect of the present disclosure, an electronic device is provided, and the electronic device includes: at least one processor; and a memory communicatively connected to the at least one processor, where the memory stores instructions executable by the at least one processor, and the instructions, when executed by the at least one processor, cause the at least one processor to perform the method as described in any of the implementations of the first aspect.

According to a fourth aspect of the present disclosure, a non-transitory computer readable storage medium storing computer instructions is provided, and the computer instructions are used to cause a computer to perform the method as described in any of the implementations of the first aspect.

According to a fifth aspect of the present disclosure, a computer program product including a computer program is provided, where the computer program, when executed by a processor, implements the method as described in any of the implementations of the first aspect.

It should be understood that the content described in this section is not intended to identify key or important features of the embodiments of the present disclosure, nor is it intended to limit the scope of the present disclosure. Other features of the present disclosure will be easily understood by the following description.

BRIEF DESCRIPTION OF THE DRAWINGS

The accompanying drawings are used for better understanding of the present solution, and do not constitute a limitation to the present disclosure.

Fig. 1 is a flowchart of an embodiment of a method for predicting an epidemic situation according to the present disclosure;

Fig. 2 shows a schematic structural diagram of a SIR-X model;

Fig. 3 is a flowchart of another embodiment of the method for predicting an epidemic situation according to the present disclosure;

Fig. 4 is a schematic structural diagram of an embodiment of an apparatus for predicting an epidemic situation according to the present disclosure; and

Fig. 5 is a block diagram of an electronic device used to implement the method for predicting an epidemic situation according to an embodiment of the present disclosure.

DETAILED DESCRIPTION OF EMBODIMENTS

The following describes example embodiments of the present disclosure in conjunction with the accompanying drawings, where various details of the embodiments of the present disclosure are included to facilitate understanding, and should be considered as merely examples. Therefore, those of ordinary skills in the art should realize that various changes and modifications may be made to the embodiments described herein without departing from the scope and spirit of the present disclosure. Also, for clarity and conciseness, descriptions of well-known functions and structures are omitted in the following description.

It should be noted that the embodiments in the present disclosure and the features in the embodiments may be combined with each other on a non-conflict basis. The present disclosure will be described below in detail with reference to the accompanying drawings and in combination with the embodiments.

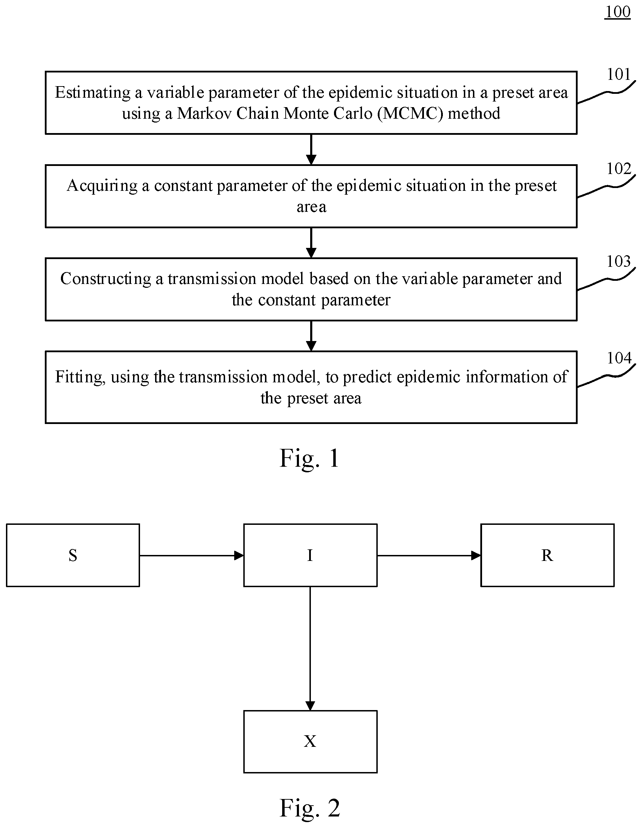

Fig. 1 shows a flow 100 of an embodiment of a method for predicting an epidemic situation according to the present disclosure. The method for predicting an epidemic situation includes the following steps 101 to 104.

Step 101 includes estimating a variable parameter of the epidemic situation in a preset area using a Markov Chain Monte Carlo (MCMC) method.

In the present embodiment, an executing body of the method for predicting an epidemic situation may use the MCMC (Markov Chain Monte Carlo) method to estimate the variable parameter of the epidemic situation in the preset area.

The epidemic situation refers to the occurrence or development of an epidemic disease, and the epidemic disease may be a large-scale infectious disease occurred in a large area or various severe infectious diseases that may occur in the future. Generally, the variable parameter of the epidemic situation may be information describing spread of the epidemic, and a value of the variable parameter changes continuously during the spread of the epidemic situation, including but not limited to at least one of: an infection speed $\alpha$ of an infected individual, an average duration $\beta\text{-}\mathbf{1}$ of infection of the infected individual, an effect $\kappa$ of a containment measure against an infective individual, an effect $\kappa_0$ of a public containment measure, or an initial number $I_0$ of the infective individual. The infection speed $\mathbf{a}$ of the infected individual may be an infection speed of an individual who contacted an infective individual. The individual who contacted the infective individual may be infected and become an infected individual, or may be not infected and recover, and $\alpha$ may be used to indicate the infection speeds of these two types of individuals. The average duration $\beta\text{-}\mathbf{1}$ of infection of the infected individual may be an average duration of infection of the individual who contacted the infective individual, and $\beta\text{-}\mathbf{1}$ may be used to indicate an average duration of infection of these two types of individuals. Generally, these two types of individuals remain infected until they recover or die. The average duration of infection of the infected individual refers to the average duration of infection of these two types of individuals before recovering or dying. The more and stricter the containment measures against the infective individual are, the greater a value of the effect $\kappa$ is. The containment measures against the infective individual may be, for example, individual monitoring and treatment, to prevent the infective individual from contacting outsiders. Similarly, the more and stricter the public containment measures are, the greater a value of the effect $\kappa_0$ is. The public containment measures may be containment measures against the general public, such as home monitoring and reducing social activities, to reduce the general public contact with outsiders. Since the epidemic situation is spread by an initial infective individual, the initial number $I_0$ of the infective individual is generally greater than zero.

The preset area may be an area of any level, including but not limited to a country, a province, a city and the like. Generally, different cities have different environments, which may cause different impacts on the spread of the epidemic situation. For example, high temperature and high humidity can significantly reduce the spread of the epidemic situation. In addition, different cities adopt different measures to contain the epidemic situation. For example, some cities adopt measures, such as staggered travel and extended statutory holidays, to reduce an inter-city population flow to reduce the spread of the epidemic situation. It can be seen that the spread of the epidemic situation in different cities is different. Furthermore, it may be concluded that distribution of the variable parameters of the epidemic situation in different cities is different. Therefore, the method for predicting an epidemic situation provided by the embodiments of the present disclosure is usually a city-level epidemic situation prediction. In other words, the preset area may be specific to a certain city.

The MCMC (Markov Chain Monte Carlo) method is a Monte Carlo method that is simulated by a computer under the framework of Bayesian theory. The method introduces a Markov process into the simulation of the Monte Carlo method, realizes a dynamic simulation in which sampling distribution changes with the simulation, and makes up for the defect that traditional Monte Carlo integration can only perform a static simulation.

Step 102 includes acquiring a constant parameter of the epidemic situation in the preset area.

In the present embodiment, the executing body may acquire the constant parameter of the epidemic situation in the preset area.

The constant parameter of the epidemic situation may be information describing an initial situation of the epidemic situation, and a value of the constant parameter is constant, including but not limited to at least one of: an initial number $S_0$ of a susceptible individual, an initial number $R_0\text{'}$ of a removal individual, or an initial number $X_0$ of a confirmed infective individual. $S_0$ is an initial value of the susceptible individual S, and is constant at zero. $R_0\text{'}$ is an initial value of the removal individual R, and is constant at zero. $X_0$ is an initial value of the confirmed infective individual X. When a time of an initial confirmed case is determined, the corresponding $X_0$ is also constant.

Generally, the population within a range of the epidemic situation may be divided into three types: type S, susceptible individuals, referring to those who are not infected but lack immune abilities and are susceptible to infection after contacting an infective individual; type I, infective individuals, referring to those who are infected with an infectious disease, and may infect an individual of the type S; and type R, removal individuals, referring to those who are individually monitored and treated, or those who have immunity due to recovery from the infection, or those who died of the infection and are not infectious any more. In addition, type I alternatively includes type X, confirmed infective individuals, referring to those who are confirmed infective individuals on the basis of clinical diagnosis.

Step 103 includes constructing a transmission model based on the variable parameter and the constant parameter.

In the present embodiment, the executing body may construct the transmission model based on the variable parameter and the constant parameter. The variable parameter and the constant parameter are used as parameters to construct a differential equation, and then the transmission model may be obtained. The transmission model may include, but is not limited to, a SIR (Susceptible Infectious Recovered) model, a SEIR (Susceptible Exposed Infectious Recovered) model, a SIR-X model, and so on. The SIR-X model is a SIR model in which an epidemic containment measure is considered.

For ease of understanding, Fig. 2 shows a schematic structural diagram of a SIR-X model. As shown in Fig. 2, the SIR-X model includes S (a susceptible individual), I (an infective individual), R (a removal individual), and X (a confirmed infective individual).

Using the SIR-X model as an example, the SIR-X model may be represented by the following differential equation:

$$\partial_t S = -\alpha SI - \kappa_0 S$$

$$\partial_t I = \alpha SI - \beta I - \kappa_0 I - \kappa I$$

$$\partial_t R = \beta I + \kappa_0 S$$

$$\partial_t X = (\kappa + \kappa_0) I$$

S is the cumulative number of the susceptible individual. *I* is the cumulative number of the infective individual. *R* is the cumulative number of the removal individual. *X* is the cumulative number of the confirmed infective individual. $\alpha$ is the infection speed of the infected individual. $\beta$-**1** is the average duration of infection of the

infected individual. $\kappa$ is the effect of the containment measure against the infective individual. $\kappa_0$ is the effect of the public containment measure. $I_0$ is the initial number of the infective individual. $S_0$ is the initial number of the susceptible individual. $R_0$' is the initial number of the removal individual. $X_0$ is the initial number of the confirmed infective individual.

Step 104 includes fitting, using the transmission model, to predict epidemic information of the preset area.

In the present embodiment, the executing body may fit, using the transmission model, to predict the epidemic information of the preset area.

The epidemic information may be used to describe characteristics of the epidemic situation, including but not limited to at least one of: a predicted average number $R_0$ of an infection caused by the infective individual, the effect $\kappa$ of the containment measure against the infective individual, the effect $\kappa_0$ of the public containment measure, the initial number $I_0$ of the infective individual, or a cumulative number $X$ of the confirmed infective individual. Generally, $R_0$ may be calculated as: $R_0 = \alpha/(\beta + \kappa + \kappa_0)$. Generally, the infective individual may cause an infection before recovering or dying of an infection.

In a specific embodiment, the transmission model may be used to fit the cumulative number $X$ of the confirmed infective individual, and then analyze a growth curve of the cumulative number $X$ of the confirmed infective individual over time, to determine whether the containment measure adopted in the preset area is effective in controlling the spread of the epidemic situation.

The method for predicting an epidemic situation provided by the embodiments of the present disclosure, first estimates a variable parameter of the epidemic situation in a preset area using a Markov Chain Monte Carlo (MCMC) method; next acquires a constant parameter of the epidemic situation in the preset area; then constructs a transmission model based on the variable parameter and the constant parameter; and finally fits, using the transmission model, to predict epidemic information of the preset area. The transmission model and the MCMC method are used to predict the epidemic situation in the preset area, which improves an accuracy of epidemic situation prediction. Using the MCMC method, an accuracy of the distribution of the estimated variable parameter of the epidemic situation is high, thereby improving an accuracy of the epidemic information fitted by the constructed transmission model. Moreover, the MCMC method can estimate the variable parameter of the epidemic situation in an area of any level (including but not limited to a country, a province, a city and the like.), so as to realize the prediction of epidemic information in an area of any level.

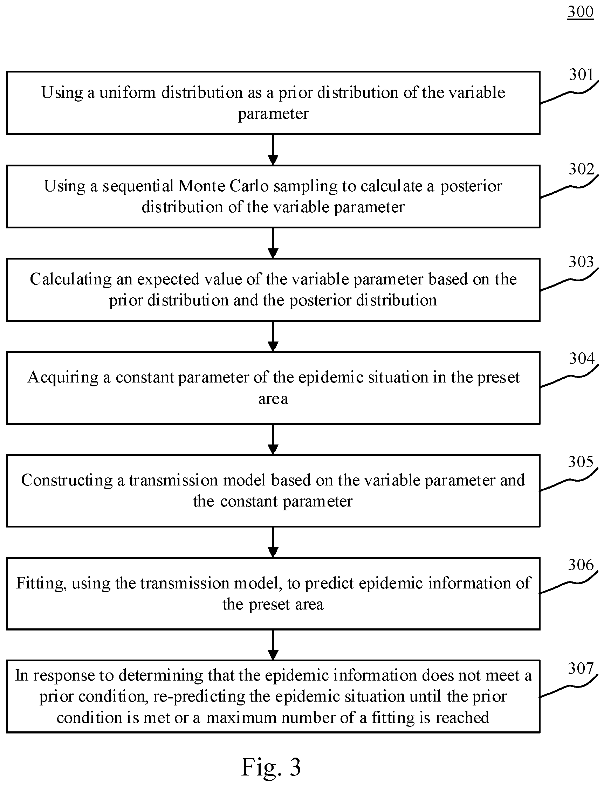

With further reference to Fig. 3, Fig. 3 shows a flow 300 of another embodiment of the method for predicting an epidemic situation according to the present disclosure. The method for predicting an epidemic situation includes the following steps 301 to 307.

Step 301 includes using a uniform distribution as a prior distribution of the variable parameter.

In the present embodiment, an executing body of the method for predicting an epidemic situation may use the uniform distribution as the prior distribution of the variable parameter of the epidemic situation.

In probability theory and statistics, the uniform distribution, also called rectangular distribution, is a symmetric probability distribution, in which the distribution probability at intervals of a given length is equally possible. The uniform distribution is defined by two parameters, a and b, which are the minimum and maximum values on a number axis, and the uniform distribution is generally abbreviated as U (a, b).

The prior distribution, also called "a distribution before an experiment" or "a previous distribution", is a kind of a probability distribution. The prior distribution is opposite to "posterior distribution", and is independent of test results or random sampling. The prior distribution reflects a distribution obtained based on knowledge of other relevant parameters before conducting statistical experiments.

Step 302 includes using a sequential Monte Carlo sampling to calculate a posterior distribution of the variable parameter.

In the present embodiment, the executing body may use the sequential Monte Carlo sampling to calculate the posterior distribution of the variable parameter of the epidemic situation.

Taking into account the nonlinearity of a transmission model, the sequential Monte Carlo sampling may be used to calculate the posterior distribution of the variable parameter of the epidemic situation. The posterior distribution may integrate relevant information provided by samples and the prior distribution, and sample to complete conversion from the prior distribution to the posterior distribution.

Step 303 includes calculating an expected value of the variable parameter based on the prior distribution and the posterior distribution.

In the present embodiment, the executing body may calculate the expected value of the variable parameter based on the prior distribution and the posterior distribution. For each variable parameter, the expected value may be a mean value of the variable parameter calculated based on the prior distribution and the posterior distribution of the variable parameter.

Step 304 includes acquiring a constant parameter of the epidemic situation in the preset area.

Step 305 includes constructing a transmission model based on the variable parameter and the constant parameter.

Step 306 includes fitting, using the transmission model, to predict epidemic information of the preset area.

In the present embodiment, specific operations of steps 304-306 are described in detail in steps 102-104 of the embodiment shown in Fig. 1, and detailed description thereof will be omitted.

Step 307 includes in response to determining that the epidemic information does not meet a prior condition, re-predicting the epidemic situation until the prior condition is met or a maximum number of the fitting is reached.

In the present embodiment, in response to determining that the epidemic information does not meet the prior condition, the executing body may return to step 301 and re-predict the epidemic situation until the prior condition is met or the maximum number of the fitting (for example, 20 times) is reached.

In a specific embodiment, if a number of the fitting does not reach the maximum number of the fitting, but epidemic information meets the prior condition, epidemic information fitted by a last constructed transmission model may be used as a final epidemic information. If a number of the fitting reaches the maximum number of the fitting, but epidemic information does not meet the prior condition, epidemic information fitted by a last constructed transmission model may still be used as the final epidemic information.

Generally, when the MCMC method is used to estimate the variable parameter of the epidemic situation in the preset area, a random number is added, so that the estimated variable parameter of the epidemic situation is different each time. Therefore, in the absence of the prior condition, results estimated using the MCMC method are not only different each time, but are also not limited. In order to make the estimated results accord with actual situations, the prior condition may be introduced. The prior condition may be a condition that limits the results estimated using the MCMC method, so that the estimated results accord with the actual situations. Generally, the prior condition may include but is not limited to at least one of: $R_0$ being smaller than the number $R_{0,free}$ of an infection caused by the infective individual without the containment measure, $\kappa_0$ being smaller than $\kappa$, $X$ being not smaller than a true cumulative number of the confirmed infective individual, or $I_0$ being greater than zero. $R_{0,free}$ represents the number of an infection without a containment measure. Generally, high humidity and high temperature can significantly reduce the spread of the epidemic situation, so $R_{0,free}$ may be different in preset areas of different geographic locations. $R_{0,free}$ represents a maximum value of $R_0$, so in actual situations, $R_0$ is smaller than $R_{0,free}$. In some embodiments, $R_{0,free}$ is 6.2, and $R_0$ is between 1.4 and 3.3. $\kappa_0$ is the effect of the public containment measure, and $\kappa$ is the effect of the containment measure against the infective individual. Since containment measures against the infective individual are more and stricter than public containment measures, in actual situations, $K_0$ is smaller than $\kappa$. Since the epidemic situation is spread by an initial infective individual, the initial number $I_0$ of the infective individual is generally greater than zero.

It can be seen from Fig. 3 that, compared with the embodiment corresponding to Fig. 1, the method for predicting an epidemic situation in the present embodiment highlights the step of estimating a variable parameter of the epidemic situation. Therefore, the solution described in the present embodiment uses the uniform distribution as the prior distribution of the variable parameter, and uses the sequential Monte Carlo sampling to calculate the posterior distribution of the variable parameter, which can take into account the nonlinearity of the SIR-X model, thereby making the calculated expected value of the variable parameter of the epidemic situation more suitable for constructing the SIR-X model.

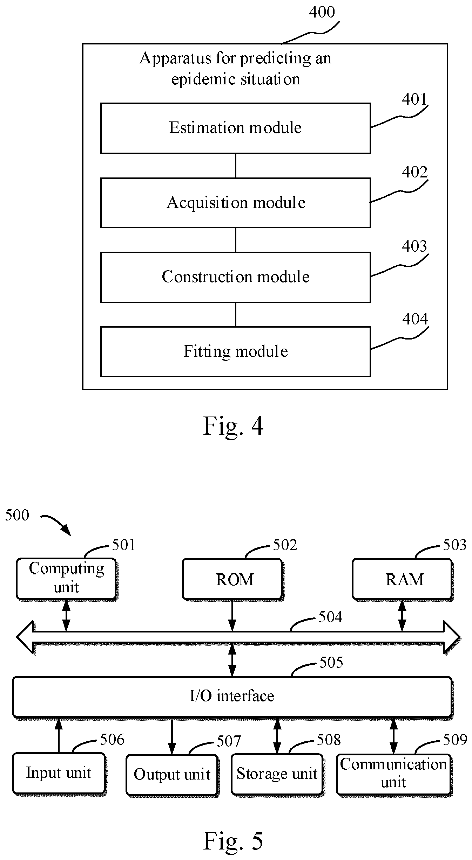

With further reference to Fig. 4, as an implementation of the method shown in the above figures, the present disclosure provides an embodiment of an apparatus for predicting an epidemic situation. The apparatus embodiment corresponds to the method embodiment as shown in Fig. 1. The apparatus may be applied to various electronic devices.

As shown in Fig. 4, an apparatus 400 for predicting an epidemic situation of the present embodiment may include: an estimation module 401, an acquisition module 402, a construction module 403 and a fitting module 404. The estimation module 401 is configured to estimate a variable parameter of the epidemic situation in a preset area using a Markov Chain Monte Carlo (MCMC) method. The acquisition module 402 is configured to acquire a constant parameter of the epidemic situation in the preset area. The construction module 403 is configured to construct a transmission model based on the variable parameter and the constant parameter. The fitting module 404 is configured to fit, using the transmission model, to predict epidemic information of the preset area.

In the present embodiment, in the apparatus 400 for predicting an epidemic situation, for the specific processing and the technical effects of the estimation module 401, the acquisition module 402, the construction module 403 and the fitting module 404, reference may be made to the relevant descriptions of steps 101-104 in the corresponding embodiment of Fig. 1 respectively, and detailed description thereof will be omitted.

In some alternative implementations of the present embodiment, the variable parameter includes at least one of: an infection speed $\alpha$ of an infected individual, an average duration $\beta$-**1** of infection of the infected individual, an effect $\kappa$ of a containment measure against an infective individual, an effect $\kappa_0$ of a public containment measure, or an initial number $I_0$ of the infective individual.

In some alternative implementations of the present embodiment, the constant parameter includes at least one of: an initial number $S_0$ of a susceptible individual, an initial number $R_0$' of a removal individual, or an initial number $X_0$ of a confirmed infective individual.

In some alternative implementations of the present embodiment, the estimation module is further configured to: use a uniform distribution as a prior distribution of the variable parameter; use a sequential Monte Carlo sampling to calculate a posterior distribution of the variable parameter; and calculate an expected value of the variable parameter based on the prior distribution and the posterior distribution.

In some alternative implementations of the present embodiment, the apparatus further includes: a re-prediction module, configured to, in response to determining that the epidemic information does not meet a prior condition, re-predict the epidemic situation until the prior condition is met or a maximum number of a fitting is reached.

In some alternative implementations of the present embodiment, the epidemic information includes at least one of: a predicted average number $R_0$ of an infection caused by the infective individual, the effect $\kappa$ of the containment measure against the infective individual, the effect $\kappa_0$ of the public containment measure, the initial number $I_0$ of the infective individual, or a cumulative number $X$ of the confirmed infective individual; and the prior condition includes at least one of: $R_0$ being smaller than the number $R_{0,free}$ of an infection caused by the infective individual without a containment measure, $\kappa_0$ being smaller than $\kappa$, $X$ being not smaller than a true cumulative number of the confirmed infective individual, or $I_0$ being greater than zero.

In some alternative implementations of the present embodiment, $R_{0,free}$ is 6.2, and $R_0$ is between 1.4 and 3.3.

According to embodiments of the present disclosure, the present disclosure further provides an electronic device, a readable storage medium and a computer program product.

Fig. 5 shows a schematic block diagram of an electronic device 500 adapted to implement embodiments of the present disclosure. The electronic device is intended to represent various forms of digital computers, such as laptops, desktops, worktables, personal digital assistants, servers, blade servers, mainframe computers and other suitable computers. The electronic device may alternatively represent various forms of mobile devices, such as personal digital processing, cellular phones, smart phones, wearable devices and other similar computing devices. The components, their connections and relationships, and their functions shown herein are examples only, and are not intended to limit the implementations of the present disclosure as described and/or claimed herein.

As shown in Fig. 5, the device 500 may include a computing unit 501, which may execute various appropriate actions and processes in accordance with a computer program stored in a read-only memory (ROM) 502 or a computer program loaded into a random-access memory (RAM) 503 from a storage unit 508. The RAM 503 may alternatively store various programs and data required by operations of the device 500. The computing unit 501, the ROM 502 and the RAM 503 are connected to each other through a bus 504. An input/output (I/O) interface 505 is also connected to the bus 504.

Multiple components of the device 500 are connected to the I/O interface 505, and include: an input unit 506, such as a keyboard and a mouse; an output unit 507, such as various types of displays and a speaker; a storage unit 508, such as a magnetic disk and an optical disk; and a communication unit 509, such as a network card, a modem and a wireless communication transceiver. The communication unit 509 allows the device 500 to exchange information or data with other devices through a computer network, such as the Internet and/or various telecommunications networks.

The computing unit 501 may be various general-purpose and/or specific-purpose processing components having processing and computing capabilities. Some examples of the computing unit 501 include, but are not limited to, a central processing unit (CPU), a graphics processing unit (GPU), various specific artificial intelligence (AI) computing chips, various computing units running machine learning model algorithms, a digital signal processor (DSP), and any appropriate processor, controller, microcontroller and the like. The computing unit 501 performs various methods and processing described above, such as the method for predicting an epidemic situation. For example, in some embodiments, the method for predicting an epidemic situation may be implemented as a computer software program, which is tangibly included in a machine-readable medium, such as the storage unit 508. In some embodiments, part or all of the computer program may be loaded and/or installed on the device 500 through the ROM 502 and/or the communication unit 509. When the computer program is loaded into the RAM 503 and executed by the computing unit 501, one or more steps of the method for predicting an epidemic situation described above may be performed. Alternatively, in other embodiments, the computing unit 501 may be configured to perform the method for predicting an epidemic situation in any other appropriate manner (such as through firmware).

The various implementations of the systems and technologies described herein may be implemented in a digital electronic circuit system, an integrated circuit system, a field programmable gate array (FPGA), an application specific integrated circuit (ASIC), an application specific standard product (ASSP), a system-on-chip (SOC), a complex programmable logic device (CPLD), computer hardware, firmware, software and/or combinations thereof. The various implementations may include: being implemented in one or more computer programs, where the one or more computer programs may be executed and/or interpreted on a programmable system including at least one programmable processor, and the programmable processor may be a specific-purpose or general-purpose programmable processor, which may receive data and instructions from a storage system, at least one input device and at least one output device, and send the data and instructions to the storage system, the at least one input device and the at least one output device.

Program codes used to implement the method of the present disclosure may be written in any combination of one or more programming languages. These program codes may be provided to a processor or controller of a general-purpose computer, specific-purpose computer or other programmable data processing apparatus, so that the program codes, when executed by the processor or controller, cause the functions or operations specified in the flowcharts and/or block diagrams to be implemented. These program codes may be executed entirely on a machine, partly on the machine, partly on the machine as a stand-alone software package and partly on a remote machine, or entirely on the remote machine or a server.

In the context of the present disclosure, the machine-readable medium may be a tangible medium that may include or store a program for use by or in connection with an instruction execution system, apparatus or device. The machine-readable medium may be a machine-readable signal medium or a machine-readable storage medium. The machine-readable medium may include, but is not limited to, an electronic, magnetic, optical, electromagnetic, infrared, or semiconductor system, apparatus or device, or any appropriate combination thereof. A more specific example of the machine-readable storage medium may include an electronic connection based on one or more lines, a portable computer disk, a hard disk, a random-access memory (RAM), a read-only memory (ROM), an erasable programmable read-only memory (EPROM or flash memory), an optical fiber, a portable compact disk read-only memory (CD-ROM), an optical storage device, a magnetic storage device, or any appropriate combination thereof.

To provide interaction with a user, the systems and technologies described herein may be implemented on a computer having: a display device (such as a CRT (cathode ray tube) or LCD (liquid crystal display) monitor) for displaying information to the user; and a keyboard and a pointing device (such as a mouse or a trackball) through which the user may provide input to the computer. Other types of devices may also be used to provide interaction with the user. For example, the feedback provided to the user may be any form of sensory feedback (such as visual feedback, auditory feedback or tactile feedback); and input from the user may be received in any form, including acoustic input, speech input or tactile input.

The systems and technologies described herein may be implemented in: a computing system including a background component (such as a data server), or a computing system including a middleware component (such as an application server), or a computing system including a front-end component (such as a user computer having a graphical user interface or a web browser through which the user may interact with the implementations of the systems and technologies described herein), or a computing system including any combination of such background component, middleware component or front-end component. The components of the systems may be interconnected by any form or medium of digital data communication (such as a communication network).

Examples of the communication network include a local area network (LAN), a wide area network (WAN), and the Internet.

A computer system may include a client and a server. The client and the server are generally remote from each other, and generally interact with each other through the communication network. A relationship between the client and the server is generated by computer programs running on a corresponding computer and having a clientserver relationship with each other.

It should be appreciated that the steps of reordering, adding or deleting may be executed using the various forms shown above. For example, the steps described in the present disclosure may be executed in parallel or sequentially or in a different order, so long as the expected results of the technical solutions provided in the present disclosure may be realized, and no limitation is imposed herein.

The above specific implementations are not intended to limit the scope of the present disclosure. It should be appreciated by those skilled in the art that various modifications, combinations, sub-combinations, and substitutions may be made depending on design requirements and other factors. Any modification, equivalent and modification that fall within the spirit and principles of the present disclosure are intended to be included within the scope of the present disclosure.

**Claims**

1. A method for predicting an epidemic situation, the method comprising:

   estimating (101) a variable parameter of the epidemic situation in a preset area using a Markov Chain Monte Carlo (MCMC) method;
   acquiring (102) a constant parameter of the epidemic situation in the preset area;
   constructing (103) a transmission model based on the variable parameter and the constant parameter; and
   fitting (104), using the transmission model, to predict epidemic information of the preset area.

2. The method according to claim 1, wherein the variable parameter comprises at least one of: an infection speed a of an infected individual, an average duration $\beta$-**1** of infection of the infected individual, an effect $\kappa$ of a containment measure against an infective individual, an effect $\kappa_0$ of a public containment measure, or an initial number $I_0$ of the infective individual.

3. The method according to claim 1 or 2, wherein the constant parameter comprises at least one of: an initial number $S_0$ of a susceptible individual, an initial number $R_0'$ of a removal individual, or an initial number $X_0$ of a confirmed infective individual.

4. The method according to any one of preceding claims, wherein estimating the variable parameter of the epidemic situation in the preset area using the Markov Chain Monte Carlo (MCMC) method, comprises:

   using (301) a uniform distribution as a prior distribution of the variable parameter;
   using (302) a sequential Monte Carlo sampling to calculate a posterior distribution of the variable parameter; and
   calculating (303) an expected value of the variable parameter based on the prior distribution and the posterior distribution.

5. The method according to any one of preceding claims, wherein the method further comprises:
   in response to determining that the epidemic information does not meet a prior condition, re-predicting (308) the epidemic situation until the prior condition is met or a maximum number of a fitting is reached.

6. The method according to any one of preceding claims, wherein the epidemic information comprises at least one of: a predicted average number $R_0$ of an infection caused by the infective individual, the effect $\kappa$ of the containment measure against the infective individual, the effect $\kappa_0$ of the public containment measure, the initial number $I_0$ of the infective individual, or a cumulative number $X$ of the confirmed infective individual; and the prior condition comprises at least one of: $R_0$ being smaller than a number $R_{0,free}$ of an infection caused by the infective individual without a containment measure, $\kappa_0$ being smaller than $\kappa$, $X$ being not smaller than a true cumulative number of the confirmed infective individual, or $I_0$ being greater than zero.

7. The method according to claim 6, wherein $R_{0,free}$ is 6.2, and $R_0$ is between 1.4 and 3.3.

8. An apparatus for predicting an epidemic situation, the apparatus comprising:

   an estimation module, configured to estimate a variable parameter of the epidemic situation in a preset area using a Markov Chain Monte Carlo (MCMC) method;
   an acquisition module, configured to acquire a constant parameter of the epidemic situation in the preset area;
   a construction module, configured to construct a transmission model based on the variable parameter and the constant parameter; and
   a fitting module, configured to fit, using the transmission model, to predict epidemic information of the preset area.

9. The apparatus according to claim 8, wherein the variable parameter comprises at least one of: an infection speed $\alpha$ of an infected individual, an average duration $\beta$-$1$ of infection of the infected individual, an effect $\kappa$ of a containment measure against an infective individual, an effect $\kappa_0$ of a public containment measure, or an initial number $I_0$ of the infective individual.

10. The apparatus according to claim 8 or 9, wherein the constant parameter comprises at least one of: an initial number $S_0$ of a susceptible individual, an initial number $R_0$' of a removal individual, or an initial number $X_0$ of a confirmed infective individual.

11. The apparatus according to any one of claims 8 to 10, wherein the estimation module is further configured to:

    use a uniform distribution as a prior distribution of the variable parameter;
    use a sequential Monte Carlo sampling to calculate a posterior distribution of the variable parameter; and
    calculate an expected value of the variable parameter based on the prior distribution and the posterior distribution

12. The apparatus according to any one of claims 8 to 11, wherein the apparatus further comprises:
    a re-prediction module, configured to, in response to determining that the epidemic information does not meet a prior condition, re-predict the epidemic situation until the prior condition is met or a maximum number of a fitting is reached.

13. The apparatus according to any one of claims 8 to 12, wherein the epidemic information comprises at least one of: a predicted average number $R_0$ of an infection caused by the infective individual, the effect $\kappa$ of the containment measure against the infective individual, the effect $K_0$ of the public containment measure, the initial number $I_0$ of the infective individual, or a cumulative number $X$ of the confirmed infective individual; and the prior condition comprises at least one of: $R_0$ being smaller than a number $R_{0,free}$ of an infection caused by the infective individual without a containment measure, $\kappa_0$ being smaller than $\kappa$, $X$ being not smaller than a true cumulative number of the confirmed infective individual, or $I_0$ being greater than zero, wherein $R_{0,free}$ is preferably 6.2, and $R_0$ is preferably between 1.4 and 3.3.

14. A non-transitory computer readable storage medium, storing computer instructions, wherein the computer instructions, when executed by a computer, cause the computer to perform the method according to any one of claims 1-7.

15. A computer program product, comprising a computer program, wherein the computer program, when executed by a processor, implements the method according to any one of claims 1-7.

100

Estimating a variable parameter of the epidemic situation in a preset area using a Markov Chain Monte Carlo (MCMC) method    101

Acquiring a constant parameter of the epidemic situation in the preset area    102

Constructing a transmission model based on the variable parameter and the constant parameter    103

Fitting, using the transmission model, to predict epidemic information of the preset area    104

Fig. 1

| S | → | I | → | R |

I → X

Fig. 2

<u>300</u>

301

Using a uniform distribution as a prior distribution of the variable parameter

302

Using a sequential Monte Carlo sampling to calculate a posterior distribution of the variable parameter

303

Calculating an expected value of the variable parameter based on the prior distribution and the posterior distribution

304

Acquiring a constant parameter of the epidemic situation in the preset area

305

Constructing a transmission model based on the variable parameter and the constant parameter

306

Fitting, using the transmission model, to predict epidemic information of the preset area

307

In response to determining that the epidemic information does not meet a prior condition, re-predicting the epidemic situation until the prior condition is met or a maximum number of a fitting is reached

Fig. 3

400

Apparatus for predicting an
epidemic situation

Estimation module — 401

Acquisition module — 402

Construction module — 403

Fitting module — 404

Fig. 4

500

Computing unit — 501

ROM — 502

RAM — 503

— 504

— 505

I/O interface

Input unit — 506

Output unit — 507

Storage unit — 508

Communication unit — 509

Fig. 5